(19) Europäisches Patentamt
European Patent Office
Office européen des brevets

(11) EP 4 525 071 A1

(12) EUROPEAN PATENT APPLICATION

(43) Date of publication:
**19.03.2025 Bulletin 2025/12**

(21) Application number: **24193464.5**

(22) Date of filing: **08.08.2024**

(51) International Patent Classification (IPC):
***H01M 4/133*** *(2010.01)* ***H01M 4/134*** *(2010.01)*
***H01M 4/136*** *(2010.01)* ***H01M 10/052*** *(2010.01)*
***H01M 10/0567*** *(2010.01)* ***H01M 10/0568*** *(2010.01)*
***H01M 10/0569*** *(2010.01)*

(52) Cooperative Patent Classification (CPC):
**H01M 10/0567; H01M 4/133; H01M 4/134; H01M 4/136; H01M 10/052; H01M 10/0568; H01M 10/0569;** H01M 2300/0028

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.08.2023 KR 20230115484**

(71) Applicant: **SAMSUNG SDI CO., LTD.**
**Giheung-gu**
**Yongin-si, Gyeonggi-do**
**17084 (KR)**

(72) Inventors:
- **Yang, Yeji**
**17084 Yongin-si, Gyeonggi-do (KR)**
- **Choi, Hyunbong**
**17084 Yongin-si, Gyeonggi-do (KR)**
- **Kim, Sundae**
**17084 Yongin-si, Gyeonggi-do (KR)**
- **Kim, Sanghoon**
**17084 Yongin-si, Gyeonggi-do (KR)**
- **Park, Sangwoo**
**17084 Yongin-si, Gyeonggi-do (KR)**
- **Sohee, Kim**
**17084 Yongin-si, Gyeonggi-do (KR)**
- **Park, Hongryeol**
**17084 Yongin-si, Gyeonggi-do (KR)**
- **Jun, Dasol**
**17084 Yongin-si, Gyeonggi-do (KR)**

(74) Representative: **Gulde & Partner**
**Patent- und Rechtsanwaltskanzlei mbB**
**Berliner Freiheit 2**
**10785 Berlin (DE)**

# (54) ELECTROLYTE OF RECHARGEABLE LITHIUM BATTERY AND RECHARGEABLE LITHIUM BATTERY INCLUDING THE SAME

(57) An electrolyte for a rechargeable lithium battery includes a lithium salt, a non-aqueous organic solvent, and an additive represented by Formula:

Formula 1

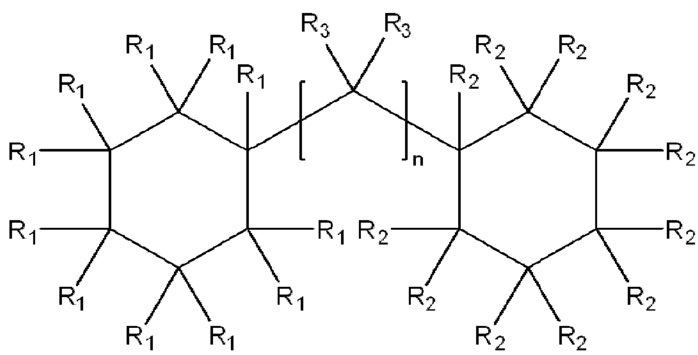

,

where at least one Ri is an isocyanate group and at least one $R_2$ is an isocyanate group.

EP 4 525 071 A1

## Description

## BACKGROUND

### 1. Field

One or more embodiments of the present disclosure relate to a rechargeable lithium battery.

### 2. Description of Related Art

Recently, with the rapid spread and popularization of battery-utilizing electronic devices, such as mobile phones, laptop computers, and/or electric vehicles, there is a rapidly increasing demand for rechargeable batteries with relatively high energy density and relatively high capacity. Therefore, intensive research has been conducted to improve performance of rechargeable lithium batteries.

A rechargeable lithium battery includes a positive electrode, a negative electrode, and an electrolyte. The positive electrode and negative electrode each include an active material capable of intercalating and de-intercalating of lithium ions, and electrical energy is generated due to oxidation and reduction reactions when lithium ions are intercalated and de-intercalated.

A lithium-transition metal oxide is utilized as a positive electrode active material of the rechargeable lithium battery. Various types (kinds) of carbon-based materials are utilized as a negative electrode active material of the rechargeable lithium battery. A lithium salt dissolved in a non-aqueous organic solvent is utilized as the electrolyte of the rechargeable lithium battery.

The rechargeable lithium battery exhibits characteristics based on complex reactions between the positive electrode and the electrolyte and between the negative electrode and the electrolyte. Accordingly, the utilization of an appropriate or suitable electrolyte is one of important variables for improvement of the rechargeable lithium battery.

## SUMMARY

The invention is defined by the appended claims. The description that follows is subjected to this limitation. Any disclosure lying outside the scope of said claims is only intended for illustrative as well as comparative purposes. One or more aspects of embodiments of the present disclosure are directed toward an electrolyte of a rechargeable lithium battery with secured safety and also with excellent or suitable battery performance.

One or more aspects of embodiments of the present disclosure are directed toward a rechargeable lithium battery including the electrolyte.

Additional aspects will be set forth in part in the description which follows and, in part, will be apparent from the description, or may be learned by practice of the presented embodiments of the disclosure

According to one or more embodiments of the present invention, an electrolyte for a rechargeable lithium battery includes a lithium salt, a non-aqueous organic solvent, and an additive represented by Formula 1.

**Formula 1**

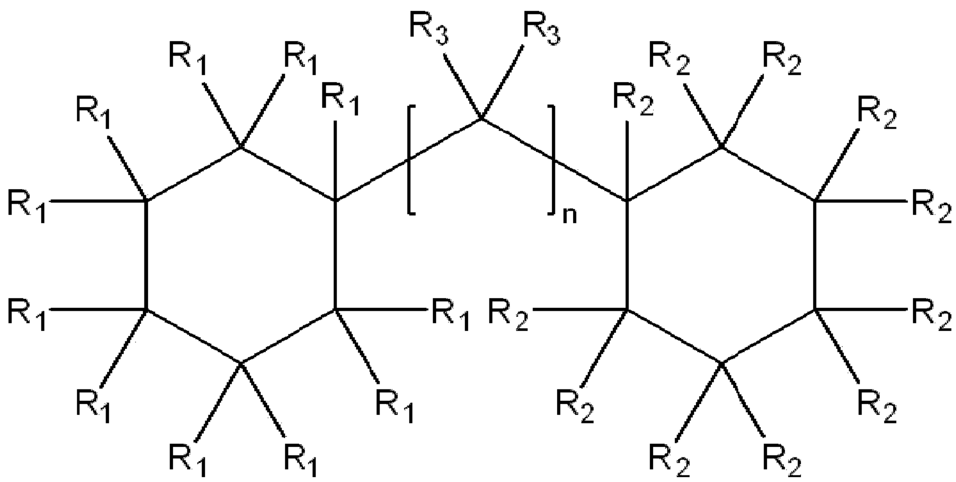

In Formula 1, $R_1$ at each occurrence are identical or different and are each independently hydrogen, a halogen, a

C1 to C10 alkyl group, or an isocyanate group. At least one $R_1$ is an isocyanate group.

In Formula 1, $R_2$ at each occurrence are identical or different and are each independently hydrogen, a halogen, a C1 to C10 alkyl group, or an isocyanate group. At least one $R_2$ is an isocyanate group.

In Formula 1, $R_3$ at each occurrence are identical or different and are each independently hydrogen or a cyclohexyl isocyanate residue.

In Formula 1, n is an integer of 1 to 10.

According to one or more embodiments of the present invention, a rechargeable lithium battery includes a positive electrode that includes a positive electrode active material, a negative electrode that includes a negative electrode active material, and the electrolyte.

## BRIEF DESCRIPTION OF DRAWINGS

The above and other aspects, features, and advantages of certain embodiments of the present disclosure will be more apparent from the following description taken in conjunction with the accompanying drawings.

FIGs. 1-5 each illustrate a simplified diagram showing a rechargeable lithium battery according to one or more embodiments of the present disclosure.

## DETAILED DESCRIPTION

In order to sufficiently understand the configuration and aspect of the present disclosure, some example embodiments of the present disclosure will be described with reference to the accompanying drawings. It should be noted, however, that the present disclosure is not limited to the described example embodiments, and may be implemented in various forms. Rather, the example embodiments are provided only to illustrate the present disclosure and let those skilled in the art fully know the scope of the present disclosure.

In the present disclosure, it will be understood that, if an element is referred to as being on another element, the element may be directly on the other element or intervening elements may be present therebetween. In the drawings, thicknesses of some components may be exaggerated for effectively explaining the technical contents. Like reference numerals refer to like elements throughout the present disclosure, and duplicative descriptions thereof is not provided for conciseness.

Example embodiments detailed in present disclosure will be discussed with reference to sectional and/or plan views as ideal exemplary views of the present disclosure. In the drawings, thicknesses of layers and regions may be exaggerated for effectively explaining the technical contents. Accordingly, regions exemplarily illustrated in the drawings have general properties, and shapes of regions exemplarily illustrated in the drawings are utilized to exemplarily disclose specific shapes but not limited to the scope of the present disclosure. It will be understood that, although the terms "first," "second," "third," etc. is utilized herein to describe various elements, these elements should not be limited by these terms. These terms are only utilized to distinguish one element from another element. The example embodiments explained and illustrated herein include complementary embodiments thereof.

The terminology utilized herein is for the purpose of describing particular embodiments only and is not intended to limit the present disclosure. As used herein, the singular forms "a," "an," and "the" are intended to include the plural forms as well, unless the context clearly indicates otherwise. Further, the utilization of "may" when describing embodiments of the present disclosure refers to "one or more embodiments of the present disclosure". As utilized herein, the terms "and/or" and "or" may include any and all combinations of one or more of the associated listed items. The terms "comprise(s)/-comprising," "Include(s)/including," and/or "have/has/having" utilized in the present disclosure do not exclude the presence or addition of one or more other components.

As utilized herein, the term "combination thereof" may refer to a mixing, a stack, a composite, a copolymer, an alloy, a blend, or a reaction product.

Unless otherwise specifically defined in the chemical formula in the present disclosure, if no chemical bond is drawn at a position where the chemical bond is supposed to be given, it means that a hydrogen atom is bonded at the position.

In the present disclosure, the term "halogen" may refer to one or more of (e.g., selected from among) F, Cl, Br, and/or I.

In the present disclosure, the term "transition metal" includes one or more of (e.g., selected from among) scandium (Sc), titanium (Ti), vanadium (V), chromium (Cr), manganese (Mn), iron (Fe), cobalt (Co), nickel (Ni), copper (Cu), zinc (Zn), yttrium (Y), zirconium (Zr), niobium (Nb), molybdenum (Mo), technetium(Tc), ruthenium (Ru), rhodium (Rh), palladium (Pd), silver (Ag), cadmium (Cd), lutetium (Lu), hafnium (Hf), tantalum (Ta), tungsten (W), rhenium (Re), osmium (Os), iridium (Ir), platinum (Pt), gold (Au), mercury (Hg), lawrencium (Lr), rutherfordium (Rf), dubnium (Db), seaborgium (Sg), bohrium (Bh), hassium (Hs), meitnerium (Mt), darmstadtium (Ds), roentgenium (Rg), and/or copernicium (Cn).

Unless otherwise specifically stated in the present specification, the expression "room temperature" means 25 ±10 °C.

In the present disclosure, the phrase "reduction in cell performance" indicates negative effects of rechargeable lithium batteries, such as a reduction in storage capacity and recovery capacity at room temperature and relatively high temperature, a reduction in capacity retention rate at 200 cycles, and an increase in amount of gas generation at relatively high-temperature storage, but the present disclosure are not limited thereto.

FIG. 1 illustrates a simplified conceptual diagram showing a rechargeable lithium battery according to one or more embodiments of the present disclosure. Referring to FIG. 1, a rechargeable lithium battery may include a positive electrode 100, a negative electrode 200, an electrolyte 300, and a separator 400.

The positive electrode 100 and the negative electrode 200 may be spaced (e.g., spaced and/or apart or spaced apart) from each other across the separator 400. The separator 400 may be between the positive electrode 100 and the negative electrode 200. The positive electrode 100, the negative electrode 200, and the separator 400 may be in contact with the electrolyte 300. The positive electrode 100, the negative electrode 200, and the separator 400 may be impregnated in the electrolyte 300.

The electrolyte 300 may be a medium through which lithium ions are transmitted between the positive electrode 100 and the negative electrode 200. The lithium ions in the electrolyte 300 may pass through the separator 400 to move toward the positive electrode 100 or the negative electrode 200. The following will describe in more detail each of the positive electrode 100, the negative electrode 200, the electrolyte 300, and the separator 400 of the rechargeable lithium battery according to one or more embodiments of the present disclosure.

**Electrolyte of Rechargeable Lithium Battery**

The electrolyte 300 for the rechargeable lithium battery includes a lithium salt, a non-aqueous organic solvent, and an additive.

The lithium salt may be dissolved in the non-aqueous organic solvent to serve as a supply source of lithium ions in the rechargeable lithium battery, and may play a role in enabling basic operations of the rechargeable lithium battery and promoting movement of lithium ions between the positive electrode and the negative electrode.

In one or more embodiments, the lithium salt may include $LiPF_6$.

The lithium salt may have a concentration of 0.1 M to 2.0 M. For example, in one or more embodiments, the lithium salt may have a concentration of equal to or greater than 0.5 M, equal to or greater than 1.0 M, or equal to or greater than 1.5 M. The lithium salt may have a concentration of equal to or less than 2.0 M, equal to or less than 1.7 M, or equal to or less than 1.5 M. When the lithium salt has a concentration of less than 0.1 M, electrolyte performance may decrease due to a reduction in electrolyte conductivity. When the lithium salt has a concentration of greater than 2.0 M, lithium ion mobility may decrease due to an increase in electrolyte viscosity.

The non-aqueous organic solvent may serve as a medium for transmitting ions that participate in an electrochemical reaction of the rechargeable lithium battery.

The non-aqueous organic solvent may include a carbonate-based solvent, an ester-based solvent, an ether-based solvent, a ketone-based solvent, an alcohol-based solvent, an aprotic solvent, or a combination thereof.

The carbonate-based solvent may include one or more of (e.g., selected from among) dimethyl carbonate (DMC), diethyl carbonate (DEC), dipropyl carbonate (DPC), methylpropyl carbonate (MPC), ethylpropyl carbonate (EPC), ethylmethyl carbonate (EMC), ethylene carbonate (EC), propylene carbonate (PC), and butylene carbonate (BC). The ester-based solvent may include one or more of (e.g., selected from among) methyl acetate, ethyl acetate, n-propyl acetate, dimethyl acetate, methyl propionate, ethyl propionate, propyl propionate, decanolide, mevalonolactone, valerolactone, and/or caprolactone. The ether-based solvent may include one or more of (e.g., selected from among) dibutyl ether, tetraglyme, diglyme, dimethoxyethane, 2-methyltetrahydrofuran, 2.5-dimethyltetrahydrofuran, and/or tetrahydrofuran. The ketone-based solvent may include cyclohexanone. The alcohol-based solvent may include one or more selected from among ethyl alcohol and isopropyl alcohol. The aprotic solvent may include nitriles such as R-CN (where, R is a hydrocarbon group having a C2 to C20 linear, branched, or cyclic structure and may include a double bond, an aromatic ring, or an ether group); amides such as dimethyl formamide; dioxolanes such as 1,3-dioxolane and/or 1.4-dioxolane; and/or sulfolanes.

The non-aqueous organic solvent may be utilized alone or in a mixture of two or more thereof.

For example, if utilized in a mixture of two or more thereof, the non-aqueous organic solvent may include one or more of carbonate-based solvents and one or more of propionate-based solvents.

The propionate-based solvent may be included in an amount of equal to or greater than 70 volume percent of a total amount (e.g., a total volume amount) of the non-aqueous organic solvent. As such, a rechargeable lithium battery may improve in high-voltage properties and/or high-temperature properties.

For example, in one or more embodiments, the non-aqueous organic solvent may be a mixed solvent of ethylene carbonate (EC), propyl carbonate (PC), and propyl propionate (PP).

In one or more embodiments, the additive may be included in an amount of 0.05 weight percent to 10 weight percent of a total amount (e.g., total weight) of the electrolyte. For example, in some embodiments, the additive may have an amount of equal to or greater than 0.1 weight percent, equal to or greater than 0.5 weight percent, equal to or greater than 1 weight percent, or equal to or greater than 5 weight percent of the total amount (e.g., total weight) of the electrolyte. The additive may have an amount of equal to or less than 8 weight percent, equal to or less than 7 weight percent, or equal to or less than 5 weight percent of the total amount (e.g., total weight) of the electrolyte. When the additive has an amount of less than 0.05 weight percent of the total amount (e.g., total weight) of the electrolyte, there may be a slight effect of solving a problem caused by transition metal ions eluted from a positive electrode current collector. When the additive has an amount of greater than 10 weight percent of the total amount (e.g., total weight) of the electrolyte, there may be a risk of reduction in lifespan at high-temperature charge/discharge cycles.

The additive according to one or more embodiments of the present disclosure is represented by Formula 1.

**Formula 1**

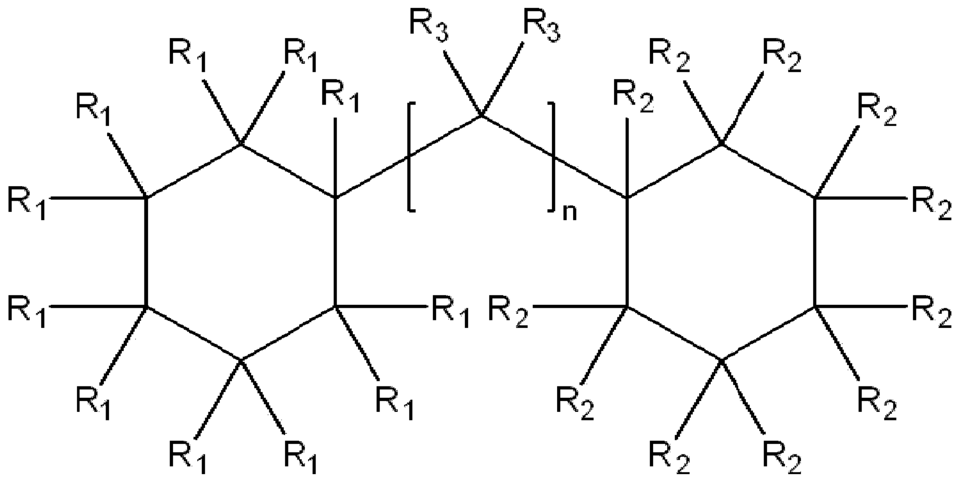

In Formula 1, $R_1$ at each occurrence are identical or different and are each independently hydrogen, a halogen, a C1 to C10 alkyl group, or an isocyanate group. At least one $R_1$ is an isocyanate group.

In Formula 1, $R_2$ at each occurrence are identical or different and are each independently hydrogen, a halogen, a C1 to C10 alkyl group, or an isocyanate group. At least one $R_2$ is an isocyanate group.

In Formula 1, $R_3$ at each occurrence are identical or different and are each independently hydrogen or a cyclohexyl isocyanate residue.

n is an integer of 1 to 10.

Although a positive electrode active material (e.g., lithium-transition metal oxide) has a stable structure, transition metal ions may be eluted during charge/discharge procedures and high-temperature storage, and side reactions may occur between the eluted transition metal ions and moisture. There may thus be a reduction in cell performance as a whole.

The electrolyte of one or more embodiments of the present disclosure to which the additive is applied may control moisture to effectively suppress or reduce transition metal ions from being eluted from a positive electrode and being precipitated on a surface of a negative electrode. Accordingly, it may effectively prevent or reduce a reduction in cell performance during charge/discharge procedures and high-temperature storage.

The additive according to one or more embodiments of the present disclosure may be represented by Formula 1-1.

**Formula 1-1**

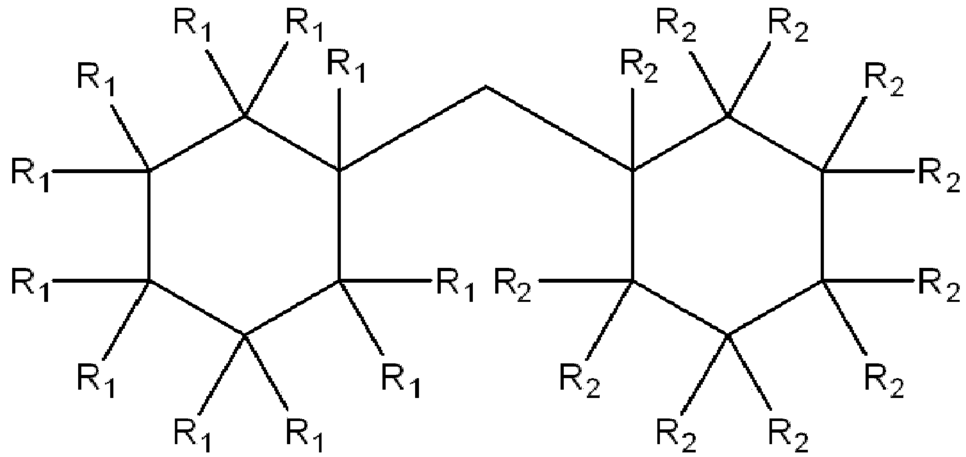

In Formula 1-1, $R_1$ at each occurrence are identical or different and are each independently hydrogen, a halogen, a C1 to C10 alkyl group, or an isocyanate group. At least one $R_1$ is an isocyanate group.

In Formula 1-1, $R_2$ at each occurrent are identical or different and are each independently hydrogen, a halogen, a C1 to C10 alkyl group, or an isocyanate group. At least one $R_2$ is an isocyanate group.

The electrolyte of one or more embodiments of present disclosure to which the additive represented by Formula 1-1 is applied may control moisture to effectively suppress or reduce transition metal ions from being eluted from a positive electrode and being precipitated on a surface of a negative electrode. Accordingly, it may effectively prevent or reduce a reduction in cell performance during charge/discharge procedures and high-temperature storage.

The additive according to one or more embodiments of the present disclosure may be represented by Formula 1-2.

**Formula 1-2**

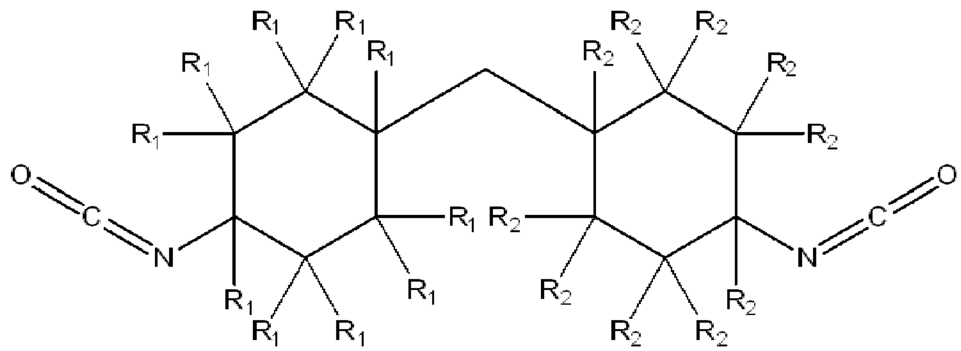

In Formula 1-2, $R_1$ at each occurrence are identical or different and are each independently hydrogen, a halogen, or a C1 to C10 alkyl group.

In Formula 1-2, $R_2$ at each occurrence are identical or different and are each independently hydrogen, a halogen, or a C1 to C10 alkyl group.

The electrolyte of one or more embodiments of the present disclosure to which the additive represented by Formula 1-2 is applied may control moisture to effectively suppress or reduce transition metal ions from being eluted from a positive electrode and being precipitated on a surface of a negative electrode. Accordingly, it may effectively prevent or reduce a reduction in cell performance during charge/discharge procedures and high-temperature storage.

In one or more embodiments, the electrolyte may further include one kind (type) of transition metal ion or a mixture of two or more kinds (types) of transition metal ions.

In one or more embodiments, the electrolyte may include one or more selected from among an iron ion, a phosphate ion, a cobalt ion, a manganese ion, a nickel ion, an aluminum ion, and a halogen ion.

In one more embodiments, the electrolyte may include an iron ion.

**Positive Electrode of Rechargeable Lithium Battery**

The positive electrode 100 of the rechargeable lithium battery may include a first current collector COL1 and a positive electrode active material layer AML1 on the first current collector COL1. The positive electrode active material layer AML1 may include a positive electrode active material and may further include one or more selected from among a binder and a conductor.

For example, in some embodiments, the positive electrode 100 may further include an additive that serves as a sacrificial positive electrode.

A content of the positive electrode active material may be in a range of 90 weight percent to 99.5 weight percent with respect to 100 weight percent of a total weight of the positive electrode active material layer AML1. Contents of the binder and the conductor may respectively be 0.5 weight percent and 5 weight percent with respect to 100 weight percent of the total weight of the positive electrode active material layer AML1.

The binder may serve to improve attachment of positive electrode active material particles to each other and also to improve attachment of the positive electrode active material to the first current collector COL1. The binder may include one or more of (e.g., selected from among) polyvinyl alcohol, carboxymethyl cellulose, hydroxypropyl cellulose, diacetyl cellulose, polyvinylchloride, carboxylated polyvinylchloride, polyvinylfluoride, ethylene oxide-containing polymer, polyvinylpyrrolidone, polyurethane, polytetrafluoroethylene, polyvinylidene fluoride, polyethylene, polypropylene, styrene-butadiene rubber, (meth)acrylated styrene-butadiene rubber, epoxy resin, (meth)acrylic resin, polyester resin, and/or nylon, but embodiments of the present disclosure are not limited thereto.

The conductor (e.g., an electrically conductive material) may be utilized to provide an electrode with conductivity, and any suitable conductive material without causing chemical change of a battery may be utilized as the conductor to constitute the battery. The conductor may include a carbon-based material such as natural graphite, artificial graphite, carbon black, acetylene black, Ketjen black, carbon fiber, carbon nano-fiber, and/or carbon nano-tube; a metal powder or fiber containing one or more of (e.g., selected from among) copper, nickel, aluminum, and/or silver; a conductive polymer such as a polyphenylene derivative; or a mixture thereof.

In one or more embodiments, the first current collector COL1 may include aluminum (Al), but embodiments of the present disclosure are not limited thereto.

**Positive Electrode Active Material**

The positive electrode active material may include a compound (e.g., a lithiated intercalation compound) that may reversibly intercalate and de-intercalate lithium. For example, in one or more embodiments, the positive electrode active material may include at least one composite oxide of lithium and metal(s) that is selected from among cobalt, manganese, nickel, and a combination thereof.

The composite oxide may include lithium transition metal composite oxides, for example, lithium-nickel-based oxides, lithium-cobalt-based oxides, lithium-manganese-based oxides, lithium-iron-phosphate-based compounds, cobalt-free nickel-manganese-based oxides, or a combination thereof.

For example, in one or more embodiments, the positive electrode active material may include a compound represented by one of the following chemical formulae : $Li_aA_{1-b}X_bO_{2-c}D_c$ ($0.90 \leq a \leq 1.8$, $0 \leq b \leq 0.5$, $0 \leq c \leq 0.05$), $Li_aMn_{2-b}X_bO_{4-c}D_c$ ($0.90 \leq a \leq 1.8$, $0 \leq b \leq 0.5$, $0 \leq c \leq 0.05$), $Li_aNi_{1-b-c}Co_bX_cO_{2-\alpha}D_\alpha$ ($0.90 \leq a \leq 1.8$, $0 \leq b \leq 0.5$, $0 \leq c \leq 0.5$, $0 < \alpha < 2$), $Li_aNi_{1-b-c}Mn_bX_cO_{2-\alpha}D_\alpha$ ($0.90 \leq a \leq 1.8$, $0 \leq b \leq 0.5$, $0 \leq c \leq 0.5$, $0 < \alpha < 2$), $Li_aNi_bCo_cL^1_dGeO_2$ ($0.90 \leq a \leq 1.8$, $0 \leq b \leq 0.9$, $0 \leq c \leq 0.5$, $0 \leq d \leq 0.5$, $0 \leq e \leq 0.1$), $Li_aNiG_bO_2$ ($0.90 \leq a \leq 1.8$, $0.001 \leq b \leq 0.1$), $Li_aCoG_bO_2$ ($0.90 \leq a \leq 1.8$, $0.001 \leq b \leq 0.1$), $Li_aMn_{1-b}G_bO_2$ ($0.90 \leq a \leq 1.8$, $0.001 \leq b \leq 0.1$), $Li_aMn_2G_bO_4$ ($0.90 \leq a \leq 1.8$, $0.001 \leq b \leq 0.1$), $Li_aMn_{1-g}G_gPO_4$ ($0.90 \leq a \leq 1.8$, $0 \leq g \leq 0.5$), $Li_{(3-f)}Fe_2(PO_4)_3$ ($0 \leq f \leq 2$), and $Li_aFePO_4$ ($0.90 \leq a \leq 1.8$).

In the foregoing chemical formulae, A may be Ni, Co, Mn, or a combination thereof; X may be Al, Ni, Co, Mn, Cr, Fe, magnesium (Mg), strontium (Sr), V, a rare-earth element, or a combination thereof; D may be oxygen (O), fluorine (F), sulfur (S), phosphorous (P), or a combination thereof; G may be Al, Cr, Mn, Fe, Mg, La, Ce, Sr, V, or a combination thereof; and $L^1$ may be Mn, Al, or a combination thereof.

For example, in one or more embodiments, the positive electrode active material may be a high-nickel-based positive electrode active material in which a content of nickel is in a range of 80 mol percent (for example, 85 mol percent, 90 mol percent, 91 mol percent, or 94 mol percent) to 99 mol percent with respect to 100 mol percent of a total metal except lithium in lithium transition metal composite oxide. The high-nickel-based positive electrode active material may achieve relatively high capacity and thus may be applied to high-density rechargeable lithium batteries.

In one or more embodiments, the positive electrode active material may include a lithium-nickel-based oxide represented by Formula 2, a lithium-cobalt-based oxide represented by Formula 3, a lithium-iron-a phosphate-based compound represented by Formula 4, a cobalt-fee lithium-nickel-manganese-based oxide represented by Formula 5, or a combination thereof.

**Formula 2** $Li_{a1}Ni_{x1}M^1_{y1}M^2_{z1}O_{2-b1}X_{b1}$

In the Formula 2, $0.9 \leq a1 \leq 1.8$, $0.3 \leq x1 \leq 1$, $0 \leq y1 \leq 0.7$, $0 \leq z1 \leq 0.7$, $0.9 \leq x1+y1+z1 \leq 1.1$, $0 \leq b1 \leq 0.1$, $M^1$ and $M^2$ may each independently be at least one element selected from among Al, B, Ba, Ca, Ce, Co, Cr, Cu, Fe, Mg, Mn, Mo, Nb, Si, Sn, Sr, Ti, V, W, and Zr, and X may be at least one element selected from among F, P, and S.

In some embodiments, in Formula 2, $0.6 \leq x1 \leq 1$, $0 \leq y1 \leq 0.4$, and $0 \leq z1 \leq 0.4$, or $0.8 \leq x1 \leq 1$, $0 \leq y1 \leq 0.2$, and $0 \leq z1 \leq 0.2$.

Formula 3 $Li_{a2}Co_{x2}M^3_{y2}O_{2-b2}X_{b2}$

In the Formula 3, $0.9 \leq a2 \leq 1.8$, $0.7 \leq x2 \leq 1$, $0 \leq y2 \leq 0.3$, $0.9 \leq x2+y2 \leq 1.1$, $0 \leq b2 \leq 0.1$, $M^3$ may be at least one element selected from among Al, B, Ba, Ca, Ce, Cr, Cu, Fe, Mg, Mn, Mo, Ni, Se, Si, Sn, Sr, Ti, V, W, Y, Zn, and Zr, and X may be at least one element selected from among F, P, and S.

**Formula 4** $Li_{a3}Fe_{x3}M^4_{y3}PO_{4-b3}X_{b3}$

In the Formula 4, $0.9 \leq a3 \leq 1.8$, $0.6 \leq x3 \leq 1$, $0 \leq y3 \leq 0.4$, and $0 \leq b3 \leq 0.1$, $M^4$ may be at least one element selected from among Al, B, Ba, Ca, Ce, Co, Cr, Cu, Mg, Mn, Mo, Ni, Se, Si, Sn, Sr, Ti, V, W, Y, Zn, and Zr, and X may be at least one element selected from among F, P, and S.

Formula 5 $Li_{a4}Ni_{x4}Mn_{y4}M^5_{z4}O_{2-b4}X_{b4}$

In the Formula 5, $0.9 \leq a2 \leq 1.8$, $0.8 \leq x4 < 1$, $0 < y4 \leq 0.2$, $0 \leq z4 \leq 0.2$, $0.9 \leq x4+y4+z4 \leq 1.1$, and $0 \leq b4 \leq 0.1$, $M^5$ may be at least one element selected from among Al, B, Ba, Ca, Ce, Cr, Fe, Mg, Mo, Nb, Si, Sn, Sr, Ti, V, W, and Zr, and X may be at least one element selected from among F, P, and S.

For example, the electrolyte according to one or more embodiments may provide an excellent or suitable improvement of reduction in cell performance of batteries to which the lithium-iron-phosphate-based oxide represented by Formula 4 is applied.

**Negative Electrode of Rechargeable Lithium Battery**

The negative electrode 200 of the rechargeable lithium battery may include a second current collector COL2 and a negative electrode active material layer AML2 on the second current collector COL2. The negative electrode active material layer AML2 may include a negative electrode active material and may further include one or more selected from among a binder and a conductor (e.g., an electron conductor).

For example, in one or more embodiments, the negative electrode active material layer AML2 may include a negative electrode active material of 90 weight percent to 99 weight percent, a binder of 0.5 weight percent to 5 weight percent, and a conductor of 0 weight percent to 5 weight percent, based on 100 weight percent of a total weight of the negative electrode active material layer.

The binder may serve to improve attachment of negative electrode active material particles to each other and also to improve attachment of the negative electrode active material to the second current collector COL2. The binder may include a non-aqueous (e.g., water-insoluble) binder, an aqueous (water-soluble) binder, a dry binder, or a combination thereof.

The non-aqueous binder may include polyvinyl chloride, carboxylated polyvinyl chloride, polyvinyl fluoride, ethylene propylene copolymer, polystyrene, polyurethane, polytetrafluoroethylene, polyvinylidene fluoride, polyethylene, polypropylene, polyamide imide, polyimide, or a combination thereof.

The aqueous binder may include styrene-butadiene rubber, (meth)acrylated styrene-butadiene rubber, (meth) acrylic rubber, butyl rubber, a fluoro elastomer, polyethylene oxide, polyvinyl pyrrolidone, polyepichlorohydrin, polyphosphazene, poly(meth)acrylonitrile, an ethylene propylene diene copolymer, polyvinyl pyridine, chlorosulfonated polyethylene, latex, a polyester resin, a (meth)acrylic resin, a phenolic resin, an epoxy resin, polyvinyl alcohol, or a combination thereof.

When an aqueous binder is utilized as the binder of the negative electrode, a cellulose-based compound may further be included, which may provide viscosity. The cellulose-based compound may include one or more of (e.g., selected from among) carboxymethyl cellulose, hydroxypropylmethyl cellulose, methyl cellulose, and/or alkaline metal salt thereof. The alkaline metal may include Na, K, or Li.

The dry binder may include a fibrillizable polymer material, for example, polytetrafluoroethylene, polyvinylidene fluoride, a polyvinylidene fluoride-hexafluoropropylene copolymer, polyethylene oxide, or a combination thereof.

The conductor (e.g., electrically conductive material) may be utilized to provide an electrode with conductivity, and any suitable conductive material without causing chemical change of a battery may be utilized as the conductor to

constitute the battery. For example, in one or more embodiments, the conductor may include a carbon-based material such as natural graphite, artificial graphite, carbon black, acetylene black, Ketjen black, carbon fiber, carbon nano-fiber, and/or carbon nano-tube; a metal powder or fiber including one or more of (e.g., selected from among) copper, nickel, aluminum, and/or silver; a conductive polymer such as a polyphenylene derivative; or a mixture thereof.

The negative electrode second current collector COL2 may include a copper foil, a nickel foil, a stainless steel foil, a titanium foil, a nickel foam, a copper foam, a polymer substrate coated with a conductive metal, or a combination thereof.

**Negative Electrode Active Material**

The negative electrode active material may include a material that may reversibly intercalate and de-intercalate lithium ions, lithium, a lithium alloy, a material that may dope and de-dope lithium, or a transition metal oxide.

The material that may reversibly intercalate and de-intercalate lithium ions may include a carbon-based negative electrode active material, for example, crystalline carbon, amorphous carbon, or a combination thereof. The crystalline carbon may include graphite, for example, natural or artificial graphite that is atypically shaped or in the shape of a plate, a flake, a sphere, or a fiber. The amorphous carbon may include, for example, soft carbon, hard carbon, mesophase pitch carbide, and/or calcined cokes.

The lithium alloy may include an alloy of lithium and a metal that is selected from among sodium (Na), potassium (K), rubidium (Rb), cesium (Cs), francium (Fr), beryllium (Be), magnesium (Mg), calcium (Ca), strontium (Sr), silicon (Si), antimony (Sb), lead (Pb), indium (In), zinc (Zn), barium (Ba), radium (Ra), germanium (Ge), aluminum (Al), and tin (Sn).

The material that may dope and de-dope lithium may include a silicon (Si)-based negative electrode active material or a tin (Sn)-based negative electrode active material. The silicon-based negative electrode active material may include silicon, a silicon-carbon composite, $SiO_x$ ($0<x\leq2$), a Si-Q alloy (where, Q is an alkali metal, an alkaline earth metal, a Group 13 element, a Group 14 element (except for Si), a Group 15 element, a Group 16 element, a transition metal, a rare-earth element, or a combination thereof), or a combination thereof. The tin-based negative electrode active material may include Sn, $SnO_k$ ($0<k\leq2$) (e.g., $SnO_2$), a Sn-based alloy, or a combination thereof.

The silicon-carbon composite may be a composite of silicon and amorphous carbon (e.g., in a form of particles). According to one or more embodiments, the silicon-carbon composite may have a structure in which amorphous carbon is coated on a surface of each of silicon particles. For example, in one or more embodiments, the silicon-carbon composite may include a secondary particle (core) where silicon primary particles are assembled and an amorphous carbon coating layer (shell) on (e.g., positioned on) a surface of the secondary particle. The amorphous carbon may also be positioned between the silicon primary particles, and for example, the silicon primary particles may be coated with the amorphous carbon. The secondary particle may be present and dispersed in an amorphous carbon matrix.

In one or more embodiments, the silicon-carbon composite may further include crystalline carbon. For example, the silicon-carbon composite may include a core including crystalline carbon and a silicon particle and an amorphous carbon coating layer positioned on a surface of the core.

In some embodiments, the silicon-based negative electrode active material and/or the tin-based negative electrode active material may be utilized by being mixed with a carbon-based negative electrode active material.

**Separator**

Based on type or kind of the rechargeable lithium battery, the separator 400 may be present between the positive electrode and the negative electrode. The separator 400 may include one or more of (e.g., selected from among) polyethylene, polypropylene, and/or polyvinylidene fluoride, or may have a multi-layered separator thereof such as a polyethylene/polypropylene bi-layered separator, a polyethylene/polypropylene/polyethylene tri-layered separator, or a polypropylene/polyethylene/polypropylene tri-layered separator.

The separator 400 may include a porous substrate and a coating layer positioned on one surface or two opposite surfaces of the porous substrate, and the coating layer may include an organic material, an inorganic material, or a combination thereof.

The porous substrate may be a polymer layer including one selected from among polyolefin such as polyethylene and/or polypropylene, polyester such as polyethylene terephthalate and/or polybutylene terephthalate, polyacetal, polyamide, polyimide, polycarbonate, polyetherketone, polyaryletherketone, polyetherimide, polyamideimide, polybenzimidazole, polyethersulfone, polyphenyleneoxide, a cyclic olefin copolymer, polyphenylenesulphide, polyethylene naphthalate, a glass fiber, and polytetrafluoroethylene (e.g., Teflon), or may be a copolymer or a mixture including two or more thereof.

The organic material may include a polyvinylidenefluoride-based copolymer and/or a (meth)acrylic copolymer.

The inorganic material may include an inorganic particle selected from among $Al_2O_3$, $SiO_2$, $TiO_2$, $SnO_2$, $CeO_2$, MgO, NiO, CaO, GaO, ZnO, $ZrO_2$, $Y_2O_3$, SrTiOs, BaTiOs, $Mg(OH)_2$, Boehmite, and/or a combination thereof, but embodiments of the present disclosure are not limited thereto.

In one or more embodiments, the organic material and the inorganic material may be present and mixed in one coating layer, or may be present in a stack form of a coating layer including the organic material and a coating layer including an inorganic material.

### Rechargeable Lithium Battery

Based on the shape of a rechargeable lithium battery, the rechargeable lithium battery according to one or more embodiments of the present disclosure may be classified into a cylinder-type or kind rechargeable lithium battery, a rectangle-type or kind rechargeable lithium battery, a pouch-type or kind rechargeable lithium battery, or a coin-type or kind rechargeable lithium battery. FIGs. 2 to 5 each illustrate a simplified diagram showing a rechargeable lithium battery according to one or more embodiments. FIG. 2 depicts a cylinder-type or kind battery, FIG. 3 depicts a rectangle-type or kind battery, and FIGs. 4 and 5 each depict a pouch-type or kind battery. Referring to FIGs. 2 to 5, a rechargeable lithium battery 1 may include an electrode assembly 500 in which a separator 400 is interposed between a positive electrode 100 and a negative electrode 200, and may also include a casing 600 in which the electrode assembly 500 is accommodated. The positive electrode 100, the negative electrode 200, and the separator 400 may be impregnated in an electrolyte. In some embodiments, the rechargeable lithium battery 1 may include a sealing member 700 that hermetically closes the casing 600 as illustrated in FIG. 2. In some embodiments, as illustrated in FIG. 3, the rechargeable lithium battery 1 may include a positive electrode lead tab 110, a positive electrode terminal 120, a negative electrode lead tab 210, and a negative electrode terminal 220. As shown in FIGs. 4 and 5, in some embodiments, the rechargeable lithium battery 1 may include an electrode tab 800, or a positive electrode tab 810 and a negative electrode tab 820, which serves as an electrical path for externally inducing a current generated in the electrode assembly 500.

The rechargeable lithium battery according to one or more embodiments of the present disclosure may be applied to automotive vehicles, mobile phones, and/or any other electrical devices, but embodiments of the present disclosure are not limited thereto.

The following will describe Embodiments and Comparatives of the present disclosure. The following Embodiments, however, are merely exemplary, and the present disclosure is not limited to the Embodiments.

### Embodiments and Comparatives

An electrolyte and a rechargeable lithium battery were fabricated by the method described herein.

### Embodiment 1

#### (1) Fabrication of Electrolyte

$LiPF_6$ of 1.15 M was dissolved in a non-aqueous organic solvent in which ethylene carbonate (EC), ethylmethyl carbonate (EMC), and dimethyl carbonate (DMC) are mixed in a volume ratio of 20:40:40, and an additive of 0.5 weight percent, based on a total weight of an electrolyte was added to fabricate the electrolyte.

A material represented by Formula 1-1-1 was utilized as the additive.

**Formula 1-1-1**

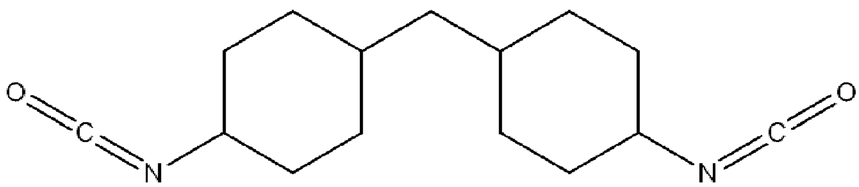

#### (2) Fabrication of Rechargeable Lithium Battery

$LiFePO_4$ as a positive electrode active material, polyvinylidene fluoride as a binder, and acetylene black as a conductor were mixed in a weight ratio of 96:3:1, and dispersed in N-methyl pyrrolidone to fabricate a positive electrode active material slurry.

The positive electrode active material slurry was coated on a 15 μm-thick Al foil, dried at 100 °C, and then pressed to fabricate a positive electrode.

Artificial graphite as a negative electrode active material, and styrene-butadiene rubber and carboxymethyl cellulose as binders were mixed in a weight ratio of 98:1:1, and dispersed in distilled water to fabricate a negative electrode

active material slurry.

The negative electrode active material slurry was coated on a 10 μm-thick Cu foil, dried at 100 °C, and then pressed to fabricate a negative electrode.

The positive electrode, the negative electrode, and a 10 μm-thick polyethylene separator were assembled to fabricate an electrode assembly, and the electrolyte was introduced into the electrode assembly to fabricate a rechargeable lithium battery.

**Embodiment 2**

An electrolyte and a rechargeable lithium battery were each fabricated in independently the same method as that in Embodiment 1, except that $LiCoO_2$ was utilized as a positive electrode active material.

**Embodiment 3**

An electrolyte and a rechargeable lithium battery were each fabricated in independently the same method as that in Embodiment 1, except that $Li(NiCoAl)O_2$ was utilized as a positive electrode active material.

**Embodiment 4**

An electrolyte and a rechargeable lithium battery were each fabricated in independently the same method as that in Embodiment 1, except that $Li(NiCoMn)O_2$ was utilized as a positive electrode active material.

**Comparative 1**

An electrolyte and a rechargeable lithium battery were each fabricated in independently the same method as that in Embodiment 1, except that no additive is added when the electrolyte was fabricated.

**Comparative 2**

An electrolyte and a rechargeable lithium battery were each fabricated in independently the same method as that in Embodiment 1, except that no additive was added when the electrolyte was fabricated and $LiCoO_2$ was utilized as a positive electrode active material.

**Comparative 3**

An electrolyte and a rechargeable lithium battery were each fabricated in independently the same method as that in Embodiment 1, except that no additive was added when the electrolyte was fabricated and $Li(NiCoAl)O_2$ was utilized as a positive electrode active material.

**Comparative 4**

An electrolyte and a rechargeable lithium battery were each fabricated in independently the same method as that in Embodiment 1, except that no additive was added when the electrolyte was fabricated and $Li(NiCoMn)O_2$ was utilized as a positive electrode active material.

**Comparative 5**

An electrolyte and a rechargeable lithium battery were each fabricated in independently the same method as that in Embodiment 1, except that a material represented by Formula 1-1-2 was utilized as an additive.

**[Formula 1-1-2]**

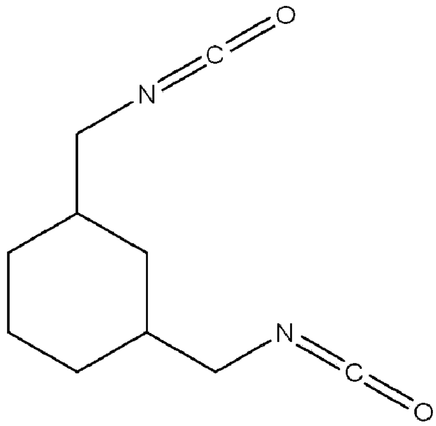

Comparative 6

An electrolyte and a rechargeable lithium battery were each fabricated in independently the same method as that in Embodiment 1, except that a material represented by Formula 1-1-3 was utilized as an additive.

**[Formula 1-1-3]**

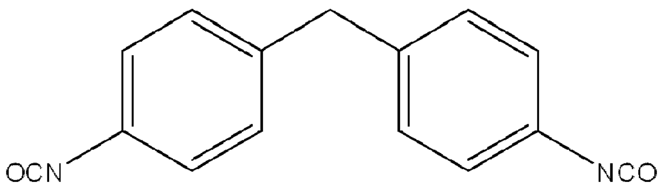

**Evaluation Example**

The following methods were used to evaluate rechargeable lithium batteries.

**Evaluation 1: Evaluation of Storage Characteristics at High Temperature (60 °C) - DCIR Increase Rate**

For each of the rechargeable lithium batteries according to Embodiments and Comparatives, after initial direct-current resistance (DCIR) was measured as $\Delta V/\Delta I$ (voltage change/current change), the battery was allowed to charge its inner maximum energy state into a full charge state (SOC 100%) and stored in the charged state at a relatively high temperature (60 °C) for 30 days, and then direct-current resistance was measured to calculate a DCIR increase rate (%) according to Equation 1 and the result was listed in Table 1.

**[Equation 1]**

$$\text{DCIR increase rate (\%)} = (\text{DCIR after 30 days / initial DCIR}) \times 100$$

[Table 1]

| Category | Initial DCIR (mΩ) | DCIR after high-temperature storage (mΩ) | DCIR increase rate after high-temperature storage (%) |
|---|---|---|---|
| Embodiment 1 | 15.15 | 15.60 | 103 |
| Embodiment 2 | 16.18 | 16.9 | 105 |
| Embodiment 3 | 16.60 | 17.76 | 107 |

(continued)

| Category | Initial DCIR (mΩ) | DCIR after high-temperature storage (mΩ) | DCIR increase rate after high-temperature storage (%) |
|---|---|---|---|
| Embodiment 4 | 16.86 | 18.2 | 108 |
| Comparative 1 | 17.29 | 20.4 | 118 |
| Comparative 2 | 17.56 | 21.07 | 120 |
| Comparative 3 | 17.30 | 21.1 | 122 |
| Comparative 4 | 17.85 | 21.95 | 123 |
| Comparative 5 | 17.96 | 22.27 | 124 |
| Comparative 6 | 18.5 | 23.86 | 129 |

**Evaluation 2: Evaluation of Charge/Discharge Characteristics at Room Temperature**

Each of the rechargeable lithium batteries according to Embodiments and Comparatives was allowed to evaluate charge/discharge characteristics at room temperature. Each of the rechargeable lithium batteries according to Embodiments and Comparatives was charged and discharged at 25 °C for 200 cycles under the condition of 0.33 C charge (CC/CV, 4.45 V, 0.025 C cut-off) and 1.0 C discharge (CC, 2.5 V cut-off).

A capacity retention rate was calculated according to Equation 2. The result was listed in Table 2.

Capacity retention rate (%) = (discharge capacity after 200 cycles/discharge capacity after 1 cycle) × 100 equation 2

[Table 2]

| | 200 Cycles at 25 °C |
|---|---|
| | Capacity retention rate (%) |
| Embodiment 1 | 92.3 |
| Embodiment 2 | 89.2 |
| Embodiment 3 | 91.6 |
| Embodiment 4 | 89.2 |
| Comparative 1 | 84.4 |
| Comparative 2 | 83.2 |
| Comparative 3 | 84.1 |
| Comparative 4 | 84 |
| Comparative 5 | 82.9 |
| Comparative 6 | 82.1 |

**Evaluation 3: Evaluation of Charge/Discharge Characteristics at High Temperature (45 °C)**

Each of the rechargeable lithium batteries according to Embodiments and Comparatives was allowed to evaluate charge/discharge characteristics at a relatively high temperature. Each of the rechargeable lithium batteries according to Embodiments and Comparatives was charged and discharged at 45 °C for 200 cycles under the condition of 0.33 C charge (CC/CV, 4.45 V, 0.025 C cut-off) and 1.0 C discharge (CC, 2.5 V cut-off).

A capacity retention rate was calculated according to Equation 2. The result was listed in Table 3.

Capacity retention rate (%) = (discharge capacity after 200 cycles/discharge capacity after 1 cycle) × 100 equation 2

[Table 3]

| | 200 Cycles at 45 °C |
|---|---|
| | Capacity retention rate (%) |
| Embodiment 1 | 89.2 |
| Embodiment 2 | 86.1 |
| Embodiment 3 | 88.5 |
| Embodiment 4 | 86.1 |
| Comparative 1 | 81.3 |
| Comparative 2 | 82.3 |
| Comparative 3 | 82.1 |
| Comparative 4 | 82.4 |
| Comparative 5 | 80.1 |
| Comparative 6 | 79.9 |

**Evaluation 4: Evaluation of Gas Generation Characteristic at High Temperature (60 °C)**

Each of the rechargeable lithium batteries according to Embodiments and Comparatives was allowed to evaluate gas generation characteristics at a relatively high temperature. The battery was allowed to charge its inner maximum energy state into a full charge state (SOC 100%) and stored in the charged state at a relatively high temperature (60 °C) for 10 or 30 days, and then a gas generation amount was evaluated and the result was listed in Table 4.

A change in volume before and after high-temperature storage was measured and the Archimedes' method was utilized to convert the change in volume into a change in mass.

[Table 4]

| | Change in amount of gas generation at 60 °C (%) | |
|---|---|---|
| | Change in amount for 10 days (%) | Change in amount for 30 days (%) |
| Embodiment 1 | 123 | 132 |
| Embodiment 2 | 124 | 133 |
| Embodiment 3 | 125 | 135 |
| Embodiment 4 | 129 | 135 |
| Comparative 1 | 137 | 146 |
| Comparative 2 | 139 | 148 |
| Comparative 3 | 138 | 147 |
| Comparative 4 | 138 | 148 |
| Comparative 5 | 141 | 153 |
| Comparative 6 | 144 | 159 |

**Comprehensive Evaluation**

Referring to Table 1, there was a dramatic increase in DCIR after high-temperature storage (60 °C) in each of the comparative examples in each of which an electrolyte to which no additive was added (Comparatives 1 to 4) was utilized, in the comparative example in which a material represented by Formula 1-1-2 as an additive (Comparative 5) was utilized, and in the comparative example in which a material represented by Formula 1-1-3 as an additive (Comparative 6) was utilized. It may thus confirm that there is a reduction in high-temperature storage characteristics of the comparative examples.

In contrast, it is ascertained that there was an improvement in DCIR increase rate even under the stand/storage at a relatively high temperature (60 °C) in each of the examples in each of which an electrolyte to which an additive according

to the present disclosure is added (Embodiments 1 to 4).

Referring to Tables 2 and 3, in each of the comparative examples in each of which an electrolyte to which no additive was added (Comparatives 1 to 4), in the comparative example in which a material represented by Formula 1-1-2 as an additive (Comparative 5) was utilized, and in the comparative example in which a material represented by Formula 1-1-3 as an additive (Comparative 6) was utilized, there was a reduction in capacity retention rate in accordance with charge/discharge cycles at room temperature and at relatively high temperature (45 °C), which resulted in a decrease in lifespan in accordance with charge/discharge cycles.

In contrast, it is ascertained that there is an improvement in capacity retention rate in accordance with charge/discharge cycles at room temperature and at relatively high temperature (45 °C) in each of the examples in each of which an electrolyte to which an additive according to the present disclosure is added (Embodiments 1 to 4).

Referring to Table 4, it is confirmed that there was a large amount of gas generation at high-temperature (60 °C) storage in each of the comparative examples in each of which an electrolyte to which no additive was added (Comparatives 1 to 4), in the comparative example in which a material represented by Formula 1-1-2 as an additive (Comparative 5) was utilized, and in the comparative example in which a material represented by Formula 1-1-3 as an additive (Comparative 6) was utilized.

In contrast, it is ascertained that gas generation at high-temperature (60 °C) storage was effectively suppressed or reduced in each of the examples in each of which an electrolyte to which an additive according to the present disclosure is added (Embodiments 1 to 4).

From the discussed evaluation results, it is ascertained that an additive according to the present disclosure is remarkably effective in preventing or reducing a reduction in cell performance.

For example, unlike Comparatives 5 and 6 where chemical stability is decreased due to relatively high reduction potential and relatively low oxidation potential, an additive according to the present disclosure may stably control moisture to effectively prevent or reduce a reduction in cell performance even if the additive includes an isocyanate group.

A rechargeable lithium battery according to one or more embodiments of the present disclosure may achieve excellent or suitable performance while securing stability.

In the present disclosure, expressions such as "at least one of," "one of," and "selected from," when preceding a list of elements, modify the entire list of elements and do not modify the individual elements of the list. For example, "at least one of a, b or c", "at least one selected from a, b, and c", "at least one selected from among a to c", etc., may indicate only a, only b, only c, both (e.g., simultaneously) a and b, both (e.g., simultaneously) a and c, both (e.g., simultaneously) b and c, all of a, b, and c, or variations thereof. The "/" utilized herein may be interpreted as "and" or as "or" depending on the situation.

In present disclosure, The term "Group" as utilized herein refers to a group of the Periodic Table of Elements according to the 1 to 18 grouping system of the International Union of Pure and Applied Chemistry ("IUPAC").

Any numerical range recited herein is intended to include all sub-ranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein and any minimum numerical limitation recited in this specification is intended to include all higher numerical limitations subsumed therein. Accordingly, Applicant reserves the right to amend this specification, including the claims, to expressly recite any sub-range subsumed within the ranges expressly recited herein.

## Claims

**1.** An electrolyte for a rechargeable lithium battery comprising:

a lithium salt;
a non-aqueous organic solvent; and
an additive represented by Formula 1,

Formula 1

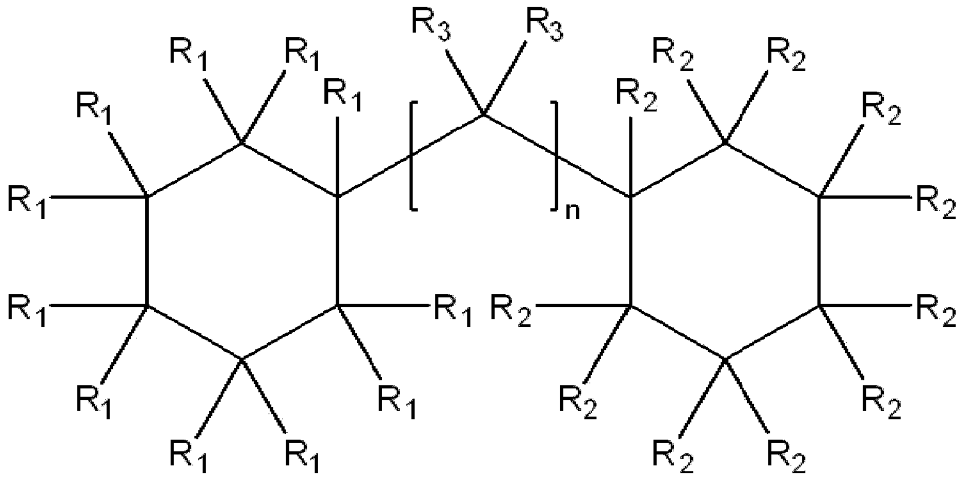

wherein, in Formula 1,

$R_1$ at each occurrence are identical or different and are each independently hydrogen, a halogen, a C1 to C10 alkyl group, or an isocyanate group, at least one $R_1$ being an isocyanate group,
$R_2$ at each occurrence are identical or different and are each independently hydrogen, a halogen, a C1 to C10 alkyl group, or an isocyanate group, at least one $R_2$ being an isocyanate group,
$R_3$ at each occurrence are identical or different and are each independently hydrogen or a cyclohexyl isocyanate residue, and
n is an integer of 1 to 10.

**2.** The electrolyte as claimed in claim 1, wherein the additive is represented by Formula 1-1,

Formula 1-1

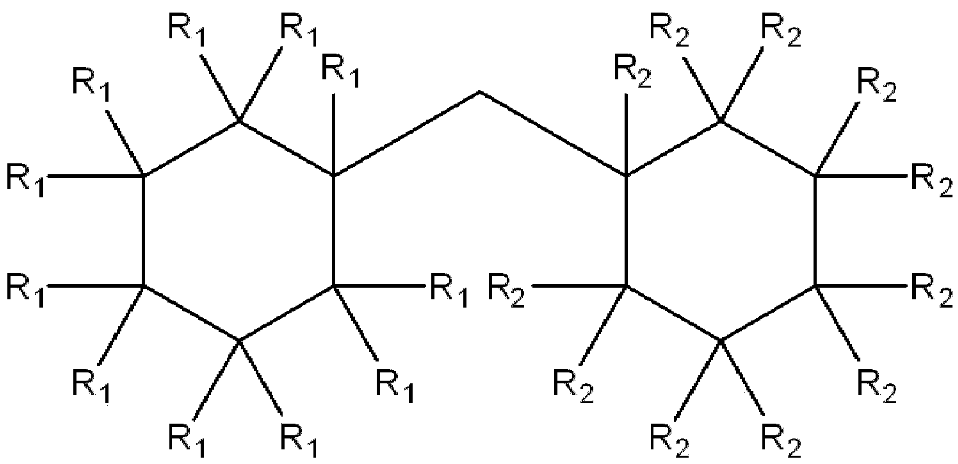

in Formula 1-1,

$R_1$ at each occurrence being identical or different and being each independently hydrogen, a halogen, a C1 to C10 alkyl group, or an isocyanate group, at least one $R_1$ being an isocyanate group, and
$R_2$ at each occurrence being identical or different and being each independently hydrogen, a halogen, a C1 to C10 alkyl group, or an isocyanate group, at least one $R_2$ being an isocyanate group.

**3.** The electrolyte as claimed in claim 1, wherein the additive is represented by Formula 1-2,

Formula 1-2

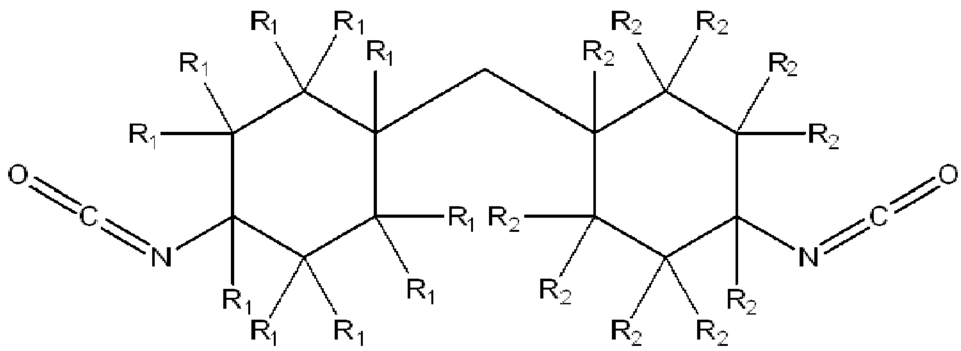

in Formula 1-2,

$R_1$ at each occurrence being identical or different and being independently hydrogen, a halogen, or a C1 to C10 alkyl group, and
$R_2$ at each occurrence being identical or different and being independently hydrogen, a halogen, or a C1 to C10 alkyl group.

**4.** The electrolyte as claimed in any one of the preceding claims, wherein the additive is in an amount of 0.05 weight percent to 10 weight percent of a total weight of the electrolyte.

**5.** The electrolyte as claimed in any one of the preceding claims, wherein the non-aqueous organic solvent comprises a carbonate-based solvent.

**6.** The electrolyte as claimed in claim 5, wherein the carbonate-based solvent comprises ethylene carbonate (EC), ethylmethyl carbonate (EMC), and dimethyl carbonate (DMC).

**7.** The electrolyte as claimed in claim 6, wherein the ethylene carbonate (EC), the ethylmethyl carbonate (EMC), and the dimethyl carbonate (DMC) are in a volume ratio of 1:(1 to 3):(1 to 3).

**8.** The electrolyte as claimed in any one of the preceding claims, wherein the lithium salt comprises $LiPF_6$.

**9.** The electrolyte as claimed in any one of the preceding claims, wherein a concentration of the lithium salt is in a range of 0.1 M to 2.0 M.

**10.** A rechargeable lithium battery, comprising:

a positive electrode (100) comprising a positive electrode active material;
a negative electrode (200) comprising a negative electrode active material; and
the electrolyte as claimed in claim 1.

**11.** The rechargeable lithium battery of claim 10, wherein the positive electrode active material comprises a lithium-iron-phosphate-based compound.

**12.** The rechargeable lithium battery of claim 10 or 11, wherein the negative electrode active material comprises a carbon-based negative electrode active material, a silicon-based negative electrode active material, a tin-based negative electrode active material, or a combination thereof.

**13.** The rechargeable lithium battery of claim 10, wherein the additive is in an amount of 0.05 weight percent to 10 weight percent of a total weight of the electrolyte.

FIG. 1

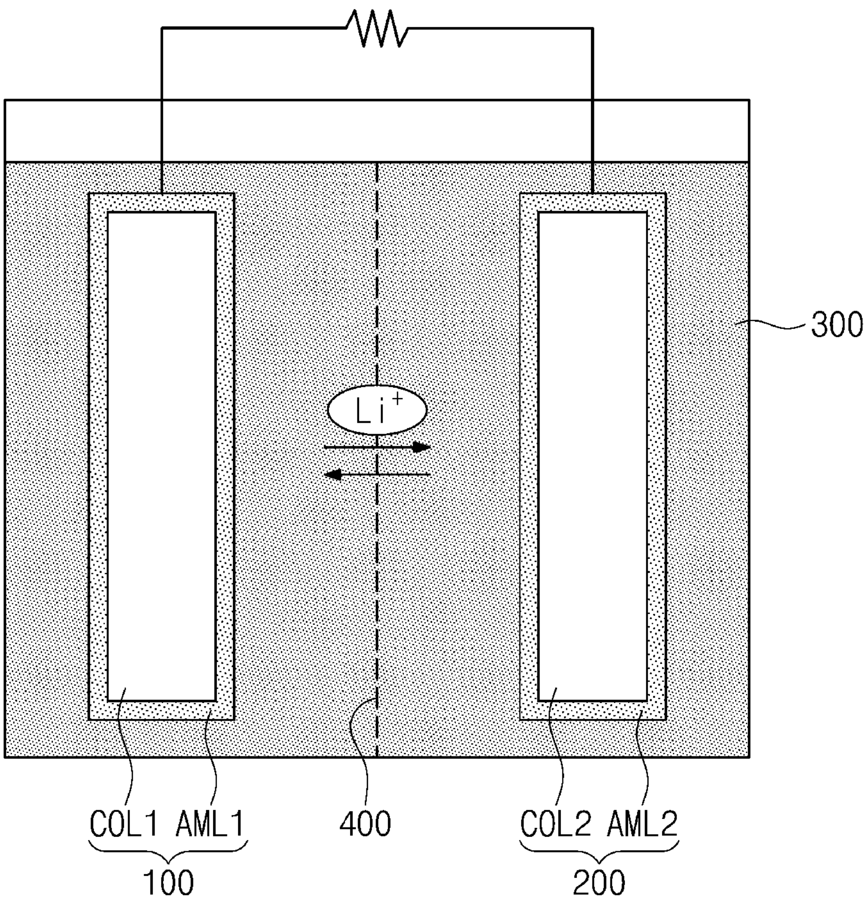

FIG. 2

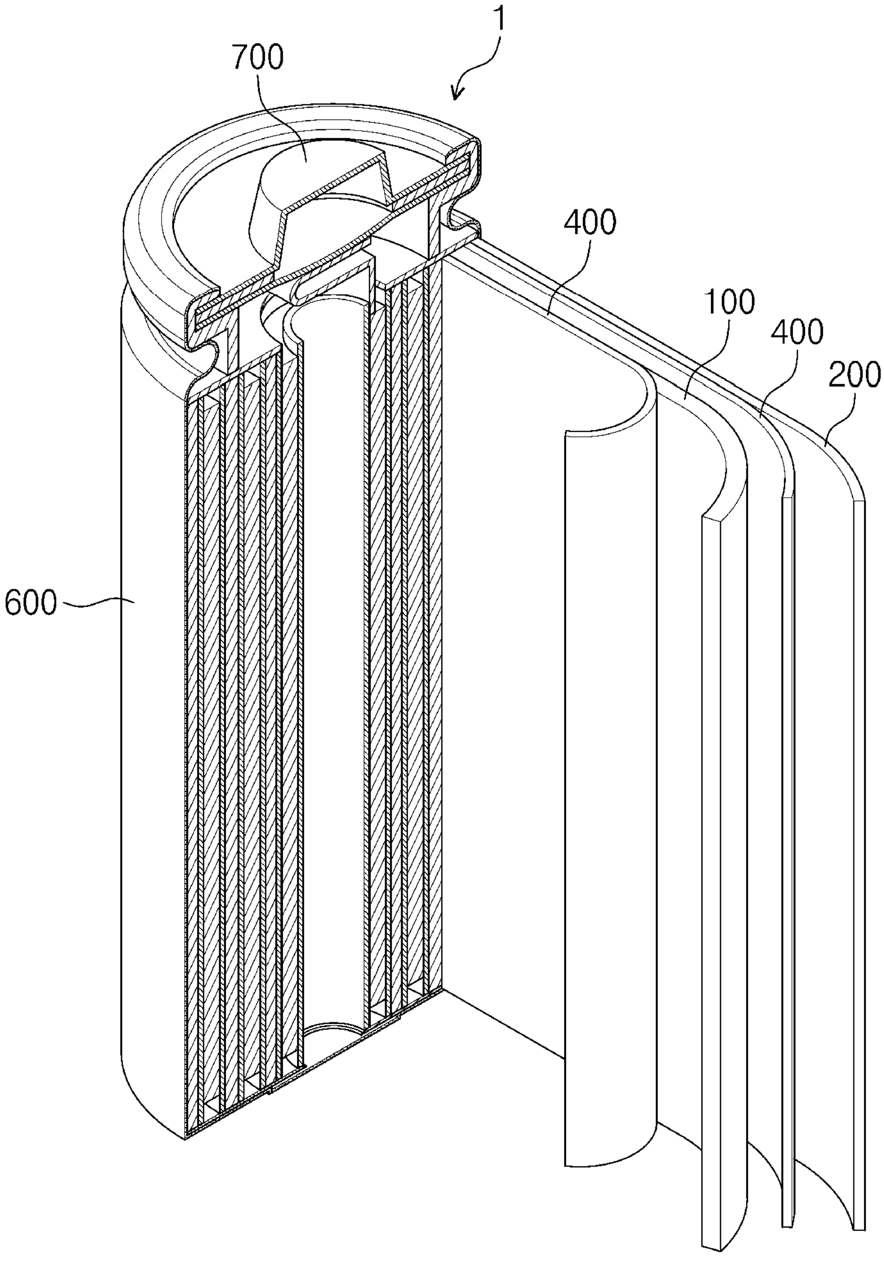

FIG. 3

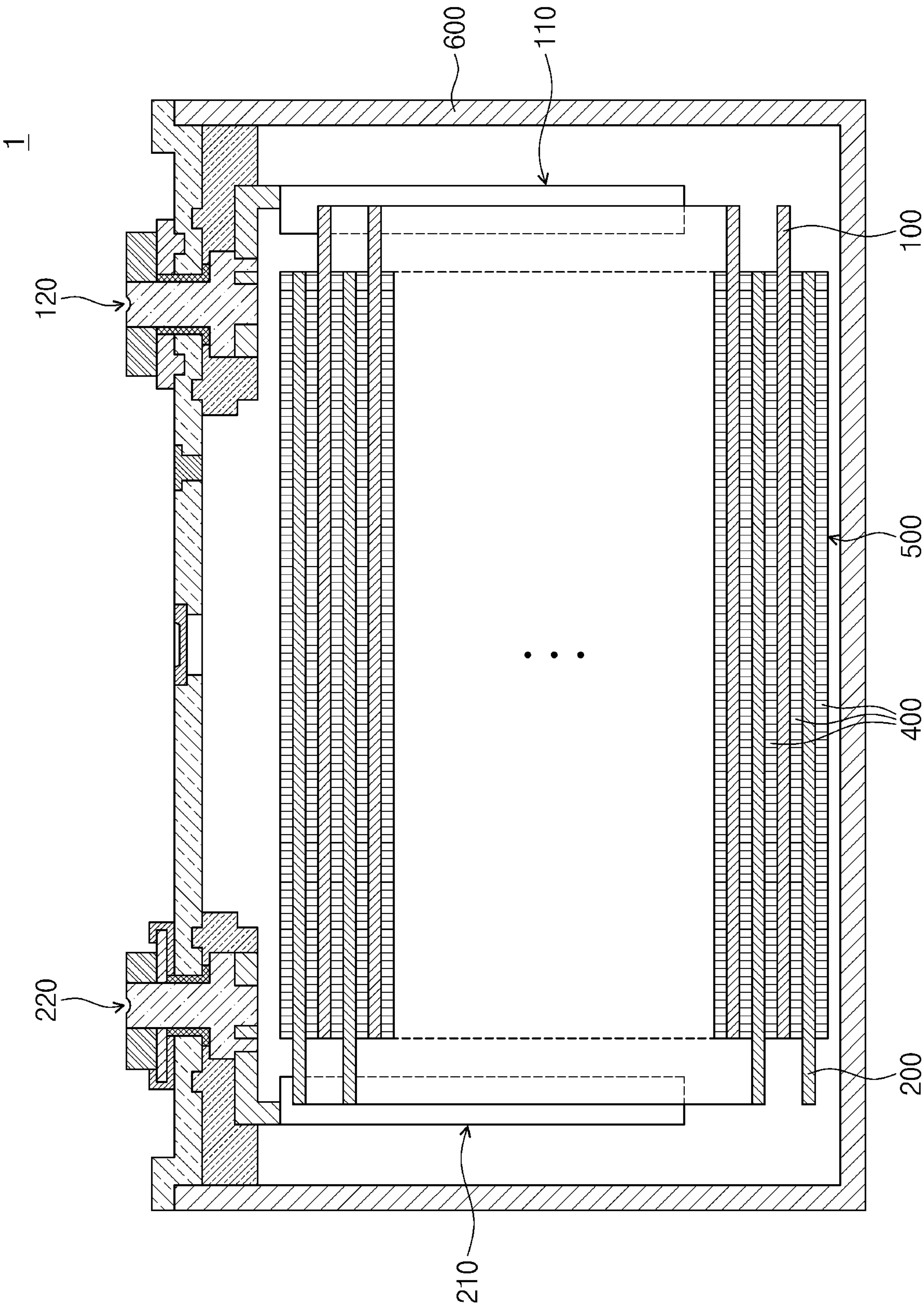

FIG. 4

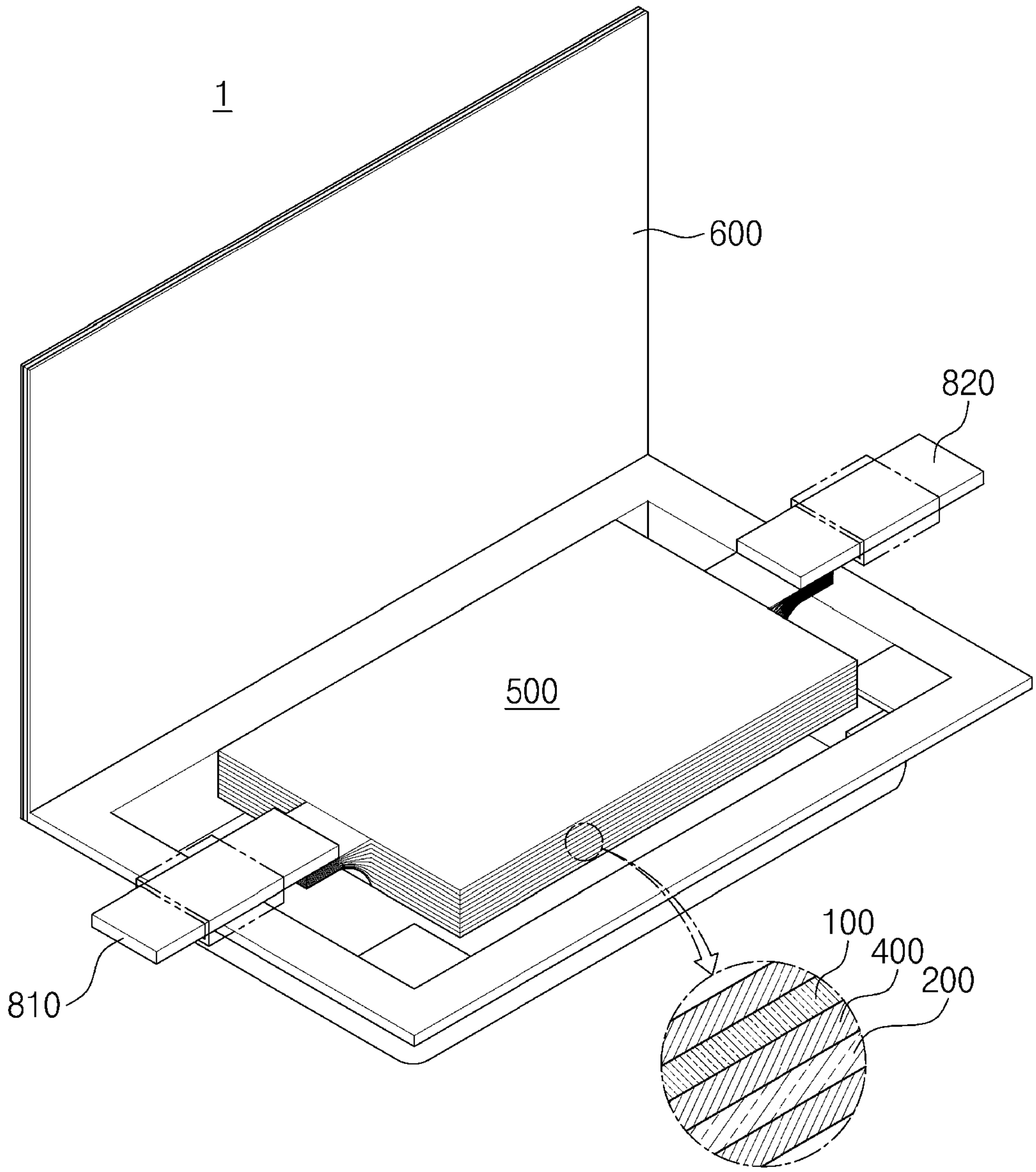

FIG. 5

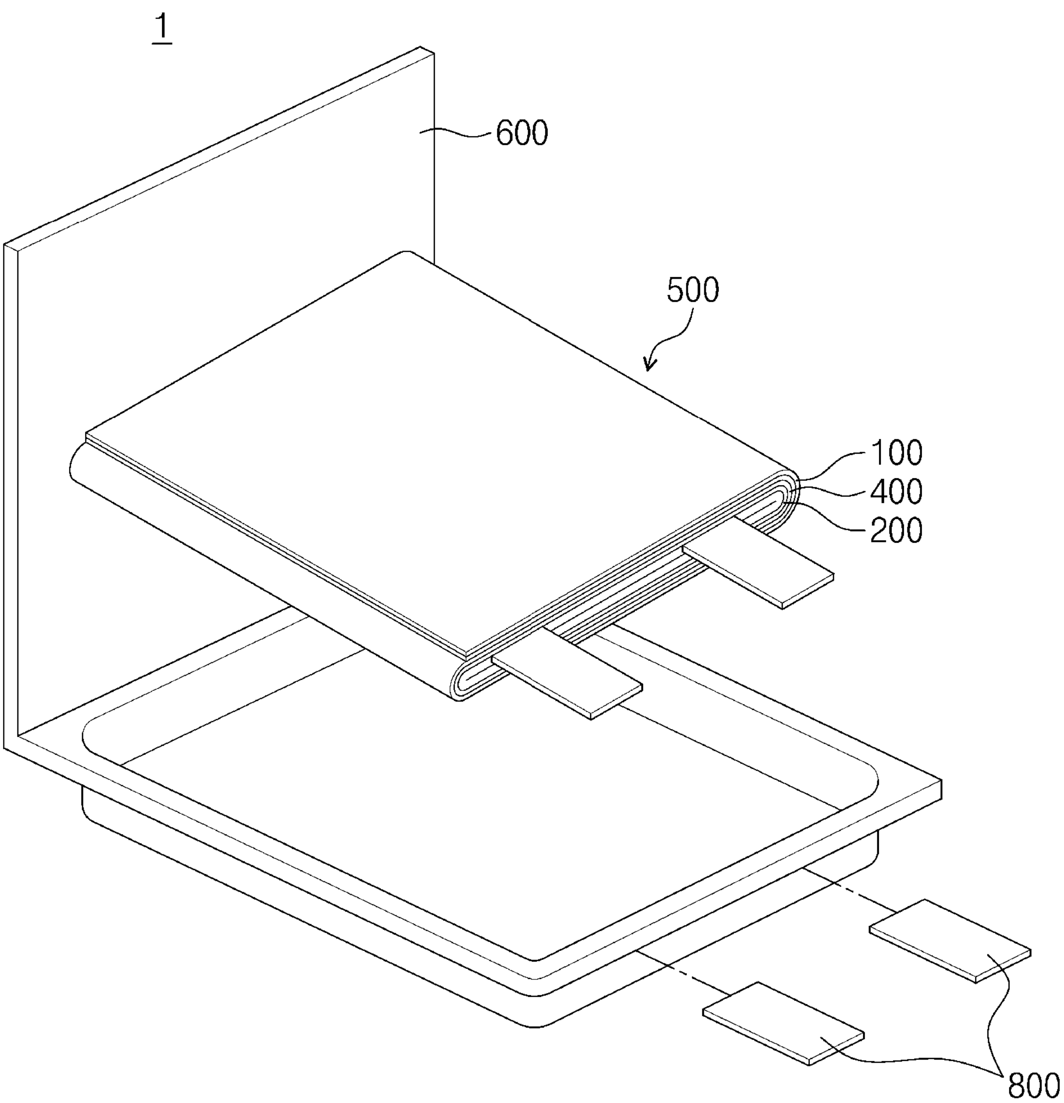

Europäisches Patentamt
European Patent Office
Office européen des brevets

# EUROPEAN SEARCH REPORT

Application Number
EP 24 19 3464

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 9 653 753 B2 (MITSUBISHI CHEM CORP [JP]) 16 May 2017 (2017-05-16) | 1-10,12, 13 | INV. H01M4/133 H01M4/134 H01M4/136 H01M10/052 H01M10/0567 H01M10/0568 H01M10/0569 |
| A | * claims 1, 18 * * examples 3-6 * * paragraph [0062] * ----- | 11 | |
| X | JP 2015 195203 A (MITSUBISHI CHEM CORP) 5 November 2015 (2015-11-05) | 1-10,12, 13 | |
| A | * abstract * ----- | 11 | |
| X | JP 5 962028 B2 (MITSUBISHI CHEM CORP) 3 August 2016 (2016-08-03) | 1-10,12, 13 | |
| A | * abstract * ----- | 11 | |

TECHNICAL FIELDS SEARCHED (IPC)

H01M

The present search report has been drawn up for all claims

| Place of search | Date of completion of the search | Examiner |
|---|---|---|
| Munich | 4 February 2025 | Koessler, Jean-Luc |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 24 19 3464

This annex lists the patent family members relating to the patent documents cited in the above-mentioned European search report. The members are as contained in the European Patent Office EDP file on
The European Patent Office is in no way liable for these particulars which are merely given for the purpose of information.

04-02-2025

| Patent document cited in search report | Publication date | Patent family member(s) | Publication date |
|---|---|---|---|
| US 9653753 B2 | 16-05-2017 | CN 103339784 A | 02-10-2013 |
| | | CN 103811815 A | 21-05-2014 |
| | | CN 109148953 A | 04-01-2019 |
| | | EP 2672561 A1 | 11-12-2013 |
| | | KR 20140036148 A | 25-03-2014 |
| | | KR 20140040284 A | 02-04-2014 |
| | | US 2013316229 A1 | 28-11-2013 |
| | | WO 2012105404 A1 | 09-08-2012 |
| JP 2015195203 A | 05-11-2015 | JP 2015195203 A | 05-11-2015 |
| | | JP 2021015812 A | 12-02-2021 |
| JP 5962028 B2 | 03-08-2016 | JP 5962028 B2 | 03-08-2016 |
| | | JP 2012178339 A | 13-09-2012 |

EPO FORM P0459

For more details about this annex : see Official Journal of the European Patent Office, No. 12/82